# EUROPEAN PATENT APPLICATION

(11) **EP 2 975 132 A1**
(43) Date of publication of application: **20.01.2016**
(21) Application number: 14177492.7
(22) Date of filing: 17.07.2014
(51) Int. Cl.: C12P 7/40, C12P 7/62, C12P 7/64

(54) **Process for producing pentanoic acid from ethanol and propionic acid**

(71) Applicant: Evonik Degussa GmbH, 45128 Essen (DE)
(72) Inventor: Haas, Thomas, 48161 Münster (DE); Beck, Simon, 48163 Münster (DE)

(57) **Abstract**

The present invention relates to a method of producing valeric acid, pentanoic acid, esters and/or salts thereof from a carbon source using at least one microorganism, wherein the carbon source is ethanol and propionic acid.

## Description

### FIELD OF THE INVENTION

The present invention is related to a biotechnological method of synthesising fatty acids. In particular, the method relates to a biotechnological method of producing at least valeric acid, heptanoic acid, esters and/or salts thereof.

### BACKGROUND OF THE INVENTION

Valeric acid, or pentanoic acid, is a straight-chain alkyl carboxylic acid with the chemical formula C₅H₁₀O₂. It is found naturally in the perennial flowering plant valerian *(Valeriana officinalis),* from which it gets its name. Its primary use is in the synthesis of its esters. Volatile esters of valeric acid tend to have pleasant odours and are used in perfumes and cosmetics. Ethyl valerate and pentyl valerate are used as food additives because of their fruity flavours (e.g. methyl valerate-flowery, ethyl valerate- fruity particularly apple, ethyl isovalerate- apple, amyl valerate- apple and pineapple). It can also be used in several applications. In particular, valeric acid, isovaleric acid and their esters are useful raw materials for a variety of industrial target compounds including plasticizers, lubricants, biodegradable solvents, lubricants, engineering plastics, epoxy curing agents, adhesive and powder coatings, corrosion inhibitors, electrolytes, vinyl stabilizers, and as an agricultural chemical intermediate. Valeric acid and esters thereof may also be used in pharmaceuticals.

Valeric acid appears similar in structure to γ-Hydroxybutyric acid (GHB), also known as 4-hydroxybutanoic acid, and the neurotransmitter γ-Aminobutyric acid /GABA) in that it is a short-chain carboxylic acid, although it lacks the alcohol and amine functional groups that contribute to the biological activities of GHB and GABA, respectively. It differs from valproic acid simply by lacking a 3-carbon side-chain.

Heptanoic acid also called enanthic acid is an organic compound composed of a seven-carbon chain terminating in a carboxylic acid. It is an oily liquid which is only slightly soluble in water, but very soluble in ethanol and ether. Heptanoic acid is usually produced to be used in the form of esters primarily for industrial lubricants due to its good corrosion properties and unique performance level at both high and low temperatures (refrigeration lubricants, aviation, automobile etc.) It can also be used in the form of esters in the flavours and fragrances industry, and in cosmetics. In the form of salts (sodium heptanoate) it is used for corrosion inhibition. Heptanoic acid can also be used to esterify steroids in the preparation of drugs such as testosterone enanthate, trenbolone enanthate, drostanolone enanthate and methenolone enanthate (Primobolan). It is also one of many additives in cigarettes.

Accordingly, valeric acid, heptanoic acid, salts and esters thereof are very useful in our day to day world. Currently, the methods of producing these carboxylic acids are strenuous and inefficient. For example, the methyl ester of ricinoleic acid, obtained from castor bean oil is the main commercial precursor to heptanoic acid. It is hydrolysed to the methyl ester of undecenoic acid and heptanal, which is then air oxidized to the carboxylic acid. This method is inefficient and results in low yields.

Some methods use petroleum based intermediates where usually cracking gasoline or petroleum is carried out. This is bad for the environment. Also, since the costs for these acids will be linked to the price of petroleum, with the expected increase in petroleum prices in the future, these acid prices may also increase relative to the increase in the petroleum prices.

There is also a method disclosed in Kenealy, W.R., 1985 where valeric acid is formed from propanol in or without the presence of ethanol. However, significant amounts of by-products such as acetic acid, butyric acid and the like are formed in the process. The yield of valerate and/or heptanoate formed in the process was also very low making it inefficient and possibly unreliable for large scale production.

Accordingly, it is desirable to find other methods of producing valeric acid, heptanoic acid, salts and esters from more sustainable raw materials, other than purely petroleum based raw materials including synthesis gas which also cause less damage to the environment.

### DESCRIPTION OF THE INVENTION

The present invention provides a biotechnological process of producing a carboxylic acid, esters and/or salts thereof from renewable fuels. In particular, the method of the present invention may comprise at least a step of converting synthesis gas to at least one carboxylic acid, esters and/or salts thereof using at least a microorganism wherein the carboxylic acid may be valeric acid, and/or pentanoic acid.

According to one aspect of the present invention, there is provided a method of producing at least one carboxylic acid, esters and/or salts thereof from a carbon source using a microorganism, wherein the carboxylic acid is valeric acid, and/or pentanoic acid.

The carbon source can be in its simplest form as carbon dioxide or carbon monoxide. In particular, the carbon source may be any complex molecule with carbon in it. More in particular, the carbon source may be selected from the group consisting of alcohols, aldehydes, glucose, sucrose, fructose, dextrose, lactose, xylose, pentose, polyol, hexose, ethanol and synthesis gas. Even more in particular, the carbon source may be a combination of ethanol and/or at least one propionate. Compared to methods known in the art, the method according to any aspect of the present invention may be able to use cheaper carbon sources such as propionate and ethanol to produce significantly higher yields of valeric acid, heptanoic acid, salts and/or esters thereof.

The combination of ethanol and/or propionic acid may be in the ratio of 1:1, 2:1, 2.1:1, 2.5:1, 3;1 and the like. More in particular the ratio of ethanol and/or propionic acid may be 2.13:1. A skilled person would understand that propionic acid may be present in its ester form in the reaction mixture.

In one example, the carbon source is ethanol and/or at least one propionate and the microorganism may be any microorganism that is capable of producing valeric acid, and/or pentanoic acid using the ethanol-carboxylate fermentation pathway. The ethanol-carboxylate fermentation pathway is described in detail at least in Seedorf, H., et al., 2008. The organism may be selected from the group consisting of *Clostridium kluyveri, C.Carboxidivorans* and the like. These microorganisms include microorganisms which in their wild-type form do not have an ethanol-carboxylate fermentation pathway, but have acquired this trait as a result of genetic modification. In particular, the microorganism may be *Clostridium kluyveri.*

The microorganism according to any aspect of the present invention may be a genetically modified microorganism. The genetically modified cell or microorganism may be genetically different from the wild type cell or microorganism. The genetic difference between the genetically modified microorganism according to any aspect of the present invention and the wild type microorganism may be in the presence of a complete gene, amino acid, nucleotide etc. in the genetically modified microorganism that may be absent in the wild type microorganism. In one example, the genetically modified microorganism according to any aspect of the present invention may comprise enzymes that enable the microorganism to produce at least one carboxylic acid. The wild type microorganism relative to the genetically modified microorganism of the present invention may have none or no detectable activity of the enzymes that enable the genetically modified microorganism to produce at least one carboxylic acid. As used herein, the term 'genetically modified microorganism' may be used interchangeably with the term 'genetically modified cell'. The genetic modification according to any aspect of the present invention is carried out on the cell of the microorganism.

The phrase "wild type" as used herein in conjunction with a cell or microorganism may denote a cell with a genome make-up that is in a form as seen naturally in the wild. The term may be applicable for both the whole cell and for individual genes. The term "wild type" therefore does not include such cells or such genes where the gene sequences have been altered at least partially by man using recombinant methods.

A skilled person would be able to use any method known in the art to genetically modify a cell or microorganism. According to any aspect of the present invention, the genetically modified cell may be genetically modified so that in a defined time interval, within 2 hours, in particular within 8 hours or 24 hours, it forms at least twice, especially at least 10 times, at least 100 times, at least 1000 times or at least 10000 times more carboxylic acid and/or the respective carboxylic acid ester than the wild-type cell. The increase in product formation can be determined for example by cultivating the cell according to any aspect of the present invention and the wild-type cell each separately under the same conditions (same cell density, same nutrient medium, same culture conditions) for a specified time interval in a suitable nutrient medium and then determining the amount of target product (carboxylic acid) in the nutrient medium.

In one example, the microorganism may be a wild type organism that expresses at least one enzyme selected E₁ to E₁₀, wherein E₁ is an alcohol dehydrogenase (adh), E₂ is an acetaldehyde dehydrogenase (aid), E₃ is an acetoacetyl- CoA thiolase (thl), E₄ is a 3-hydroxybutyryl-CoA dehydrogenase (hbd), E₅ is a 3-hydroxybutyryl-CoA dehydratase (crt), E₆ is a butyryl-CoA dehydrogenase (bcd), E₇ is an electron transfer flavoprotein subunit (etf), E₈ is a coenzyme A transferase (cat), E₉ is an acetate kinase (ack) and E₁₀ is phosphotransacetylase (pta). In particular, the wild type microorganism according to any aspect of the present invention may express at least E₂, E₃ and E₄. Even more in particular, the wild type microorganism according to any aspect of the present invention may express at least E₄.

In another example, the microorganism according to any aspect of the present invention may be a genetically modified organism that has increased expression relative to the wild type microorganism of at least one enzyme selected E₁ to E₁₀, wherein E₁ is an alcohol dehydrogenase (adh), E₂ is an acetaldehyde dehydrogenase (ald), E₃ is an acetoacetyl-CoA thiolase (thl), E₄ is a 3-hydroxybutyryl-CoA dehydrogenase (hbd), E₅ is a 3-hydroxybutyryl-CoA dehydratase (crt), E₆ is a butyryl-CoA dehydrogenase (bcd), E₇ is an electron transfer flavoprotein subunit (etf), E₈ is a coenzyme A transferase (cat), E₉ is an acetate kinase (ack) and E₁₀ is phosphotransacetylase (pta). In particular, the genetically modified microorganism according to any aspect of the present invention may express at least enzymes E₂, E₃ and E₄. Even more in particular, the genetically modified microorganism according to any aspect of the present invention may express at least E₄. The enzymes E₁ to E₁₀ may be isolated from *Clostridium kluyveri.*

The phrase "increased activity of an enzyme", as used herein is to be understood as increased intracellular activity. Basically, an increase in enzymatic activity can be achieved by increasing the copy number of the gene sequence or gene sequences that code for the enzyme, using a strong promoter or employing a gene or allele that codes for a corresponding enzyme with increased activity and optionally by combining these measures. Genetically modified cells or microorganisms used in the method according to the invention are for example produced by transformation, transduction, conjugation or a combination of these methods with a vector that contains the desired gene, an allele of this gene or parts thereof and a vector that makes expression of the gene possible. Heterologous expression is in particular achieved by integration of the gene or of the alleles in the chromosome of the cell or an extrachromosomally replicating vector.

The cells according to any aspect of the present invention are genetically transformed according to any method known in the art. In particular, the cells may be produced according to the method disclosed in WO/2009/077461.

The phrase 'the genetically modified cell has an increased activity, in comparison with its wild type, in enzymes'as used herein refers to the activity of the respective enzyme that is increased by a factor of at least 2, in particular of at least 10, more in particular of at least 100, yet more in particular of at least 1000 and even more in particular of at least 10000.

According to any aspect of the present invention, E₁ may be an ethanol dehydrogenase. In particular, E₁ may be selected from the group consisting of alcohol dehydrogenase 1, alcohol dehydrogenase 2, alcohol dehydrogenase 3, alcohol dehydrogenase B and combinations thereof. More in particular, E₁ may comprise sequence identity of at least 50% to a polypeptide selected from the group consisting of CKL_1075, CKL_1077, CKL_1078, CKL_1067, CKL_2967, CKL_2978, CKL_3000, CKL_3425, and CKL_2065. Even more in particular, E₁ may comprise a polypeptide with sequence identity of at least 50, 60, 65, 70, 75, 80, 85, 90, 91, 94, 95, 98 or 100% to a polypeptide selected from the group consisting of CKL_1075, CKL_1077, CKL_1078 and CKL_1067.

According to any aspect of the present invention, E₂ may be an acetaldehyde dehydrogenase. In particular, E₂ may be selected from the group consisting of acetaldehyde dehydrogenase 1, alcohol dehydrogenase 2 and combinations thereof. In particular, E₂ may comprise sequence identity of at least 50% to a polypeptide selected from the group consisting of CKL_1074, CKL_1076 and the like. More in particular, E₂ may comprise a polypeptide with sequence identity of at least 50, 60, 65, 70, 75, 80, 85, 90, 91, 94, 95, 98 or 100% to a polypeptide selected from the group consisting of CKL_1074 and CKL_1076.

According to any aspect of the present invention, E₃ may be selected from the group consisting of acetoacetyl-CoA thiolase A1, acetoacetyl-CoA thiolase A2, acetoacetyl-CoA thiolase A3 and combinations thereof. In particular, E₃ may comprise sequence identity of at least 50% to a polypeptide selected from the group consisting of CKL_3696, CKL_3697, CKL_3698 and the like. More in particular, E₃ may comprise a polypeptide with sequence identity of at least 50, 60, 65, 70, 75, 80, 85, 90, 91, 94, 95, 98 or 100% to a polypeptide selected from the group consisting of CKL_3696, CKL_3697 and CKL_3698.

According to any aspect of the present invention, E₄ may be 3-hydroxybutyryl-CoA dehydrogenase 1, 3-hydroxybutyryl-CoA dehydrogenase 2 and the like. In particular, E₄ may comprise sequence identity of at least 50% to a polypeptide CKL_0458, CKL_2795 and the like. More in particular, E₄ may comprise a polypeptide with sequence identity of at least 50, 60, 65, 70, 75, 80, 85, 90, 91, 94, 95, 98 or 100% to the polypeptide CKL_0458 or CKL_2795.

According to any aspect of the present invention, E₅ may be 3-hydroxybutyryl-CoA dehydratase 1, 3-hydroxybutyryl-CoA dehydratase 2 and combinations thereof. In particular, E₅ may comprise sequence identity of at least 50% to a polypeptide selected from the group consisting of CKL_0454, CKL_2527 and the like. More in particular, E₅ may comprise a polypeptide with sequence identity of at least 50, 60, 65, 70, 75, 80, 85, 90, 91, 94, 95, 98 or 100% to a polypeptide selected from the group consisting of CKL_0454 and CKL_2527.

According to any aspect of the present invention, E₆ may be selected from the group consisting of butyryl-CoA dehydrogenase 1, butyryl-CoA dehydrogenase 2 and the like. In particular, E₆ may comprise sequence identity of at least 50% to a polypeptide selected from the group consisting of CKL_0455, CKL_0633 and the like. More in particular, E₆ may comprise a polypeptide with sequence identity of at least 50, 60, 65, 70, 75, 80, 85, 90, 91, 94, 95, 98 or 100% to a polypeptide selected from the group consisting of CKL_0455 and CKL_0633.

According to any aspect of the present invention, E₇ may be selected from the group consisting of electron transfer flavoprotein alpha subunit 1, electron transfer flavoprotein alpha subunit 2, electron transfer flavoprotein beta subunit 1 and electron transfer flavoprotein beta subunit 2. In particular, E₇ may comprise sequence identity of at least 50% to a polypeptide selected from the group consisting of CKL_3516, CKL_3517, CKL_0456, CKL_0457 and the like. More in particular, E₇ may comprise a polypeptide with sequence identity of at least 50, 60, 65, 70, 75, 80, 85, 90, 91, 94, 95, 98 or 100% to a polypeptide selected from the group consisting of CKL_3516, CKL_3517, CKL_0456 and CKL_0457.

According to any aspect of the present invention, E₈ may be coenzyme transferase (cat). In particular, E₈ may be selected from the group consisting of butyryl-CoA: acetate CoA transferase, succinyl-CoA:coenzyme A transferase, 4-hydroxybutyryl-CoA: coenzyme A transferase and the like. More in particular, E₈ may comprise sequence identity of at least 50% to a polypeptide selected from the group consisting of CKL_3595, CKL_3016, CKL_3018 and the like. More in particular, E₈ may comprise a polypeptide with sequence identity of at least 50, 60, 65, 70, 75, 80, 85, 90, 91, 94, 95, 98 or 100% to a polypeptide selected from the group consisting of CKL_3595, CKL_3016 and CKL_3018.

According to any aspect of the present invention, E₉ may be an acetate kinase A (ack A). In particular, E₉ may comprise sequence identity of at least 50% to a polypeptide sequence of CKL_1391 and the like. More in particular, E₉ may comprise a polypeptide with sequence identity of at least 50, 60, 65, 70, 75, 80, 85, 90, 91, 94, 95, 98 or 100% to a polypeptide of CKL_1391.

According to any aspect of the present invention, E₁₀ may be phosphotransacetylase (pta). In particular, E₁₀ may comprise sequence identity of at least 50% to a polypeptide sequence of CKL_1390 and the like. More in particular, E₁₀ may comprise a polypeptide with sequence identity of at least 50, 60, 65, 70, 75, 80, 85, 90, 91, 94, 95, 98 or 100% to a polypeptide of CKL_1390.

In one example the microorganism, wild-type or genetically modified expresses E₁-E₁₀. In particular, the microorganism according to any aspect of the present invention may have increased expression relative to the wild type microorganism of E₁, E₂, E₃, E₄, E₅, E₆, E₇, E₈, E₉, E₁₀ or combinations thereof. In one example, the genetically modified microorganism has increased expression relative to the wild type microorganism of E₁, E₂, E₃, E₄, E₅, E₆, E₇, E₈, E₉ and E₁₀. More in particular, a combination of any of the enzymes E₁-E₁₀ may be present in the organism to enable at least one carboxylic acid to be produced. In one example, the genetically modified organism used according to any aspect of the present invention may comprise a combination of any of the enzymes E₁-E₁₀ that enable the organism to produce at least one, or two or three types of carboxylic acids at the same time. For example, the microorganism may be able to produce hexanoic acid, butyric acid and/or acetic acid at the simultaneously. Similarly, the microorganism may be genetically modified to express a combination of enzymes E₁-E₁₀ that enable the organism to produce either a single type of carboxylic acid or a variety of carboxylic acids. In all the above cases, the microorganism may be in its wild-type form or be genetically modified.

In one example, the genetically modified microorganism according to any aspect of the present invention has increased expression relative to the wild type microorganism of hydrogenase maturation protein and/or electron transport complex protein. In particular, the hydrogenase maturation protein (hyd) may be selected from the group consisting of hydE, hydF or hydG. In particular, the hyd may comprise sequence identity of at least 50% to a polypeptide selected from the group consisting of CKL_0605, CKL_2330, CKL_3829 and the like. More in particular, the hyd used according to any aspect of the present invention may comprise a polypeptide with sequence identity of at least 50, 60, 65, 70, 75, 80, 85, 90, 91, 94, 95, 98 or 100% to a polypeptide selected from the group consisting of CKL_0605, CKL_2330 and CKL_3829.

In one example, the microorganism according to any aspect of the present invention may be capable of producing at least valeric acid, heptanoic acid, esters and/or salts thereof from a carbon source of propionate/propionic acid and ethanol. In one example, valeric acid may be produced in a concentration of 50, 60, 70, 80, 90, 95% in the reaction mixture. In another example, heptanoic acid may be produced simultaneously in the resultant mixture. In a further example, only valeric acid is formed.

Throughout this application, any data base code, unless specified to the contrary, refers to a sequence available from the NCBI data bases, more specifically the version online on 12 June 2014, and comprises, if such sequence is a nucleotide sequence, the polypeptide sequence obtained by translating the former.

According to any aspect of the present invention, the carboxylic acid ions may be optionally isolated. The valeric acid, pentanoic acid, salts and/or esters thereof can be removed from the fermentation broth, for example, by continuous extraction with a solvent. The microorganisms can also be collected, for example, by decantation or filtration of the fermentation media, and a new batch of water containing the carbon source of propionate and ethanol can be combined with the microorganism. The microorganisms may thus be recyeled.

In particular, the method according to any aspect of the present invention may comprise a step of extracting the valeric acid, pentanoic acid, salts and/or esters thereof produced from the reaction mixture using any method known in the art. In particular, one example of an extraction method of carboxylic acid is provided in section 2.3 of Byoung, S.J et al. 2013. Another example may the method disclosed under the section 'Extraction Model' in Kieun C., et al., 2013.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

### Clostridium kluyveri forming valeric acid and heptanoic acid from propionic acid and ethanol

For the biotransformation of ethanol and propionic acid to valeric acid and heptanoic acid the bacterium *Clostridium kluyveri* was used. All cultivation steps were carried out under anaerobic conditions in pressure-resistant glass bottles that can be closed airtight with a butyl rubber stopper.

For the preculture 100 ml of DMSZ52 medium (pH = 7.0; 10 g/L K-acetate, 0.31 g/L K₂HPO₄, 0.23 g/L KH₂PO₄, 0.25 g/l NH₄Cl, 0.20 g/l MgSO₄x7 H₂O, 1 g/L yeast extract, 0.50 mg/L resazurin, 10 µl/l HCl (25%, 7.7 M), 1.5 mg/L FeCl₂x4H₂O, 70 µg/L ZnCl₂x7H₂O, 100 µg/L MnCl₂x4H₂O, 6 µg/L H₃BO₃, 190 µg/L CoCl₂x6H₂O, 2 µg/L CuCl₂x6H₂O, 24 µg/L NiCl₂x6H₂O, 36 µg/L Na₂MO₄x2H₂O, 0.5 mg/L NaOH, 3 µg/L Na₂SeO₃x5H₂O, 4 µg/L Na₂WO₄x2H₂O, 100 µg/L vitamin B12, 80 µg/L p-aminobenzoic acid, 20 µg/L D(+) Biotin, 200 µg/L nicotinic acid, 100 µg/L D-Ca-pantothenate, 300 µg/L pyridoxine hydrochloride, 200 µg/l thiamine -HClx2H₂O, 20 ml/L ethanol, 2.5 g/L NaHCO₃, 0.25 g/L cysteine-HClxH₂O, 0.25 g/L Na₂Sx9H₂O) in a 250 ml bottle were inoculated with 5 ml of a frozen cryoculture of *Clostridium kluyveri* and incubated at 37°C for 119 h to an OD₆₀₀ₙₘ >0.2.

For the main culture 200 ml of fresh DMSZ52 medium in a 500 ml bottle were inoculated with centrifuged cells from the preculture to an OD₆₀₀ₙₘ of 0.1. This growing culture was incubated at 37°C for 21 h to an OD₆₀₀ₙₘ >0.4. Then the cell suspension was centrifuged, washed with production buffer (pH 6.0; 1.0 g/L propionic acid, 2.5 g/l ethanol) and centrifuged again.

For the production culture, 200 ml of production buffer in a 500 ml bottle was inoculated with the washed cells from the main culture to an OD₆₀₀ₙₘ of 0.2. The culture was capped with a butyl rubber stopper and incubated for 68 h at 37°C and 100 rpm in an open water shaking bath. At the start and end of the culturing period, samples were taken. These were tested for optical density, pH and the different analytes (tested by NMR).

The results showed that in the production phase the amount of propionic acid decreased from 1.08 g/l to 0.04 g/l and the amount of ethanol decreased from 2.5 g/l to 1.5 g/l. Also, the concentration of valeric acid was increased from 0.05 g/l to 0.95 g/l and the concentration of heptanoic acid was increased from 0.00 g/l to 0.29 g/l.

## Claims

1. A method of producing valeric acid, pentanoic acid, esters and/or salts thereof from a carbon source using at least one microorganism, wherein the carbon source is ethanol and propionic acid.

2. The method according to claim 1, wherein the microorganism is capable of producing the carboxylic acid using ethanol-carboxylate fermentation.

3. The method according to claim 2, wherein the microorganism is selected from the group consisting of *Clostridium kluyveri* and C. *Carboxidivorans.*

4. The method according to either claim 2 or 3, wherein the microorganism expresses at least one enzyme selected from the group consisting of alcohol dehydrogenase E₁, acetaldehyde dehydrogenase E₂, acetoacetyl-CoA thiolase E₃, 3-hydroxybutyryl-CoA dehydrogenase E₄, 3-hydroxybutyryl-CoA dehydratase E₅, butyryl-CoA dehydrogenase E₆, electron transfer flavoprotein subunit E₇, coenzyme A transferase E₈, acetate kinase E₉ and phosphotransacetylase E₁₀.

5. The method according to claim 4, wherein the microorganism expresses E₁, E₂, E₃, E₄, E₅, E₆, E₇, E₈, E₉ and E₁₀.

6. The method according to any one of claims 2 to 5, wherein the microorganism expresses hydrogenase maturation protein and/or electron transport complex protein.

7. The method according to any one of claims 2 to 6, wherein the microorganism is genetically modified and the genetically modified microorganism has increased expression relative to the wild type microorganism of at least one enzyme selected from the group consisting of E₁, E₂, E₃, E₄, E₅, E₆, E₇, E₈, E₉, E₁₀, hydrogenase maturation protein and/or electron transport complex protein.

8. The method according to claim 7, wherein the genetically modified microorganism has increased expression relative to the wild type microorganism of E₁, E₂, E₃, E₄, E₅, E₆, E₇, E₈, E₉ and E₁₀.

9. An isolated valeric acid, pentanoic acid, esters and/or salts thereof produced according to the method of any one of the claims 1 to 8.
